# EUROPEAN PATENT APPLICATION

(11) **EP 3 017 785 A1**
(43) Date of publication of application: **11.05.2016**
(21) Application number: 13888587.6
(22) Date of filing: 01.07.2013
(51) Int. Cl.: A61C 8/00, A61C 13/225

(54) **SYSTEM FOR SECURING A DENTAL PROSTHESIS**

(71) Applicant: Martinez Navarro, Manuel Angel, 29720 Malaga (ES)
(72) Inventor: Martinez Navarro, Manuel Angel, 29720 Malaga (ES)
(74) Representative: Carvajal y Urquijo, Isabel
(86) International application number: PCT/ES2013/070447
(87) International publication number: WO 2015/001137

(57) **Abstract**

The invention relates to a system for securing a dental prosthesis, characterized in that it has at least one anchorage (1) in the form of a screw which is anchored in a single bone a distance from the maxillary alveolar ridge, and a plate (3) arranged in the oral cavity (2), under the mucosa that covers the jaw bone (4), in contact with said jaw bone (4), and following the shape thereof and of the mucosa, with an extension (5) whereby the plate (3) is connected to the anchorage, said connection being completely covered by the mucosa and being a distance from the maxillary alveolar ridge (1). There are also posts (6) in the plate (3) configured for going through the mucosa so as to secure the dental prosthesis. The loads resulting from the use of the dental prosthesis are thus transmitted to the anchorage (1) through the plate and posts (3).

## Description

### Field of the Invention

The present invention belongs to the technical field of dental prostheses, specifically to the means for securing dental prostheses, and more specifically to the means of anchoring dental prostheses in the bone in cases of a jaw bone loss, insufficient jaw bone, or serious bone atrophy. The invention particularly relates to a system for securing a complete or partial dental prosthesis with anchorages that are anchored in a single bone a distance from the maxillary alveolar ridge, which can be the zygomatic bone, or the upper or lower jaw bone, and plates arranged under the mucosa that covers the jaw bone, adapted to the shape of the bone, with an extension whereby they are firmly connected to said anchorage, said connection being completely covered by the mucosa and a distance from the maxillary alveolar ridge.

The dental prosthesis is secured to the plate by means of posts arranged in the plate in an ideal position for positioning the dental prosthesis, which posts go through the mucosa that covers the alveolar ridge of the jaw bone.

By means of this configuration, the loads resulting from the use of the dental prosthesis are transmitted to the anchorages through the plate.

### Background of the Invention

Missing teeth have serious repercussions on the chewing function, the articulation of words and aesthetics. In cases of serious bone loss in jaw bones, conventional implants cannot be used, and it is necessary to resort to special techniques. These techniques primarily consist of bone grafts for recovering bone loss, alveolar bone distraction osteogenesis, lifting of the maxillary sinus with bone filling, lateralization of the mandibular dental nerve, corticotomy and alveolar expansion, with or without graft. All these techniques have a series of drawbacks, such as, among others, being complex techniques that are prolonged in time, because it is necessary to wait at least six months until insertion of the implants. Furthermore, they present a morbidity rate when the bone must be obtained from another donor area of the individual, and they show frequent risks of infection and failure of the method.

Subsequently, Professor Branemark developed implants special known as zygomatic implants requiring a high degree of specialization by the dentist or surgeon performing implantation, since they are difficult to place and are not free of risks and issues that will be described below. These zygomatic implants solved the issue of those patients with such significant bone atrophy or lack of bone that it does not allow placing conventional or standard implants, or due to cancer or trauma, but only in the upper jaw, not in the lower jaw or mandible. These zygomatic implants are anchored in two different bones, i.e., an apical portion is anchored in the zygomatic bone, and the other oral portion is anchored in the upper jaw, going through the maxillary sinus and emerging directly into the mouth in the palate, where it is connected directly to the dental prosthesis.

Zygomatic implants only exist for the upper jaw and are mainly indicated for bilateral or serious partial maxillary atrophy, to prevent the morbidity of autologous bone grafts, in atrophy of the poster sectors, in full atrophies as a substitute for reconstruction with grafts, and in partial or complete defects in the upper jaw due to cancer or trauma.

Said zygomatic implants have the following drawbacks:
- The technique for insertion of such implants is very complicated and a high degree of specialization is required to that end, and therefore the professionals placing these implants require a very long learning curve.
- Anesthesia is general for the most part, or more rarely local anesthesia with sedation, and the operation must be performed in a hospital operating room.
- The zygomatic implant is introduced from the palate, going through the maxillary sinus and reaching the zygomatic bone through its inner face, which is an extremely complicated operation that will furthermore present great discomfort and possible subsequent complications for the patient.
- The hole for inserting the implant is made blindly, without seeing the inner face of the zygomatic bone, which is where said implant is introduced.
- In that area, irrigation does not reach the drill making the hole where the implant is arranged, with the subsequent risk of the bone becoming overheated.
- The zygomatic implant is introduced in the zygomatic bone and in contact with said bone only in one portion, and not in its entirety, this portion generally entailing at most a third of the total length of the implant, which results in a relatively small osseointegration surface, with the securing issues this entails.
- Given that implants go through the maxillary sinus, complications such as sinusitis and oral sinus fistulas often occur.
- The jaw bone is very thin on many occasions, and therefore fundamental anchorage is based on the zygomatic bone, which is usually more than three centimeters away from the end where the teeth are anchored and in an almost horizontal position or in a very oblique position, where significant tangential forces occur, rather axial forces or longitudinal forces with respect to the major axis of the implant, as would be advisable.
- Zygomatic implants emerge into the mouth through the palate, instead of emerging through the alveolar ridge, which would be the most physiological solution, and hence where they are connected directly to the dental prosthesis, causing discomfort due to the volume projecting into the palate. This discomfort ranges from discomfort due to the bulge in the mouth, issues with pronunciation, and inflammation of the mucosa surrounding the implant, given that it has a quality different from the mucosa in the gum, also resulting in issues for suitable hygiene in that area.
- The connection is contaminated with germs from the mouth so there is a risk of infection at the level of the anchorage (peri-implantitis), which can lead to the loss thereof.
- Serious complications, such as invasion of the orbital cavity and the cranial cavity while placing the implant, have been described on occasion.
- In complete rehabilitations of the upper portion, conventional implants are required in the front portion; many times there is not enough bone in that area and the method must be completed with bone grafts, which was precisely what was sought to be avoided with these zygomatic implants. Finally, the placement of more than one zygomatic implant on each side, and therefore not having to perform grafts in the front portion, has been proposed for these cases, but there is still no clinical data or sufficient studies offering long-term support for this technique, and in any case this variant even further complicates the surgical technique, which was already complex in and of itself for securing a reduced number of implants.
- These zygomatic implants are not applicable in an atrophic mandible.
- They are also not applicable in cases of serious atrophy of the molar areas of the jaw.
- Zygomatic implants are not applicable in cases of a thin ridge (very thin alveolar bone) in both the upper jaw and lower jaw or mandible either. In said cases, the only alternatives are bone grafts, dental nerve lateralization, alveolar distraction osteogenesis, and the placement of implants between the mental foramen and the prosthesis made with an extension or cantilever. The drawbacks of these techniques are, as previously stated, in some cases morbidity of the required surgery and the prolonged time for completing treatment, and in other cases the risk of fracture of the prosthesis.
- They also fail to resolve cases of the loss of a complete fragment of the mandible due to cancer or trauma, the only option for which today for rehabilitating with secured teeth are microvascularized bone graft techniques, which are very complex techniques and which, for the most part, do not provide optimal and conditions for the purpose that is sought.

A system is therefore desirable for securing a dental prosthesis, which allows securing teeth for both the upper jaw and the lower jaw, in cases of bone loss, insufficient bone, or serious bone atrophy, avoiding the drawbacks found in systems existing today in the state of the art.

### Description of the Invention

The present invention solve the issues existing in the state of the art by means of a system for securing a dental prosthesis having as a first component at least one anchorage in the form of a screw configured for being anchored in a single bone a distance from the maxillary alveolar ridge, which is located and emerge in areas other than those of conventional implants. As a second component, a plate, structure or mesh configured for being arranged in the oral cavity, under the mucosa that covers the jaw bone, in contact with the jaw bone, and following the shape thereof and of the mucosa. The plate has an extension whereby it is connected to the anchorages, said connection being completely covered by the mucosa and a distance from the maxillary alveolar ridge.

The system furthermore has as a third component a plurality of posts or attachments arranged in the plate and configured for going through the mucosa that covers the jaw bone in an optimal position so as to secure the dental prosthesis to said posts.

The loads resulting from the use of the dental prosthesis are transmitted to the anchorages through the plate.

The anchorages are elements preferably made in the form of a screw which are secured in the bone where they will become osseointegrated in a rigid manner, and will serve as support for holding the rest of the required elements, plate and posts, for being able to secure a dental prosthesis in cases of a lack of bone, insufficient or scarcity of bone.

The point where these anchorages have the connection, where they are secured to the plate or structure, unlike systems existing in the state of the art, is fully covered by tissues or mucosa, and does not emerge directly into the mouth but rather is done a distance from the maxillary alveolar ridge.

This system for securing has the advantage that the connection to the plate that will support the dental prosthesis is completely covered by the mucosa and a distance from the maxillary alveolar ridge without emerging directly into the mouth, and therefore does not become contaminated with germs from the mouth, so there is no risk of infection at the level of the anchorage (peri-implantitis), which can lead to the loss of the anchorage, and prevents the oral sinus fistula that sometimes occurs with conventional zygomatic implants, because the point of connection does not open into the gum and because in no case does it go through the maxillary sinus like in the state of the art in the case of zygomatic implants

Another advantage of this system is to prevent the bothersome bulge in the palate as a result of the connection to zygomatic implant in the state of the art, preventing issues with pronunciation, retention of bacterial plaque due to poor access and therefore poor hygiene, and therefore inflammation of the mucosa surrounding the implant. This is achieved because by means of this system, the anchorage is not connected directly to the prosthesis, but rather is connected to the plate, said plate being what in turn receives the prosthesis through the posts that will be arranged in the ideal position for supporting the prosthesis under the best conditions.

The teeth of the prosthesis will therefore have a better arrangement in the mouth because the plate or structure is custom fitted to the arch of the gum and there is no bulge through the palate. Therefore, the loads produced due to chewing and the use of the dental prosthesis are transmitted from the prosthesis through the plate to the anchorages, thereby preventing the possibility of breaking the anchorages since if there are excessive loads, they are absorbed by the prosthesis or the plate.

In the present invention, the anchorage is placed in a single bone and a distance from the maxillary alveolar ridge. The zygomatic implant of the state of the art, however, is based on an anchorage in two bones, the zygomatic bone and the upper jaw, which are separated by the maxillary sinus, which the anchorage must go through, and, since the maxillary sinus is a cavity existing in the mouth in contact with the nasal cavity and in which there are germs, it causes a significant percentage of cases of sinusitis after the placement of zygomatic implants.

By means of the system object of the present invention, the anchorage does not in any case have to go through the maxillary sinus, completely eliminating the risk of sinusitis.

Another issue arising with zygomatic implants results from the fact that the anchorage must be placed from the palate to the zygomatic bone, seeing the point of insertion into the jaw bone but not the point of insertion into the zygomatic bone, because it is introduced through its inner face. For this reason it is a very technically difficult and precise insertion; it is done blindly, with the risk of perforating the orbital cavity, and even the cranial cavity, so zygomatic implants today are normally placed with general anesthesia in a hospital. The system object of the present invention solves the issues of the state of the art in the case of zygomatic implants which are secured to two bones, given that the anchorages are only secured to one bone and a distance from the maxillary alveolar ridge (usually the zygomatic bone, although they could be fixed in the upper jaw, or in the lower jaw or mandible, on a case-to-case basis), access to the point of insertion into this bone is simple and the point of insertion can be seen perfectly, since it is inserted through the outer and lower face and not the inner face of the zygomatic bone. Therefore, it is technically much easier to place these anchorages and the aforementioned risks are avoided. Furthermore, the learning curve of the surgeon who will implant the system will be much smaller, and the system can be implanted with anesthesia local in any clinic. This will all result in a more widespread use of this system by implantologists, odontologists and surgeons, given that the technique is greatly simplified and therefore there will be many more patients with serious issues of atrophy or lack of bone in the jaw bones who would benefit from this system and could have a permanent set of teeth.

Furthermore, by means of the system object of the present invention, the external irrigation on the drill for making the hole goes directly to the bone, preventing the risk of the bone becoming overheated and therefore the risk of non-osseointegration of the implant. As stated above, according to different embodiments of the system for securing object of the present invention, the anchorages can be configured for being anchored only to the zygomatic bone from its lower edge, sagittally through same, i.e., from bottom to top, or for being anchored only to the upper jaw bone, axially through same, i.e., from its outer face to its inner face, or only to the lower jaw bone, axially through same, i.e., from its outer face to its inner face, and sagittally, i.e., from top to bottom through same, from its top edge.

Issues of lack of or atrophy of bone in the mandible can be solved by means of the securing of the anchorages to the lower jaw bone or mandible. Long, complicated techniques from the current state of the art, which have added morbidity, such as bone grafts, lateralization of the dental nerve, or bone osteogenesis, will therefore be avoided.

Additionally, in cases of the complete loss of a fragment of mandible due to tumor resection or due to trauma, continuity of the mandibular arch is lost and the segments or stumps are spaced from one another on both sides of the defect caused. In these cases, very complex techniques involving microvascularized grafts, mandibular distraction osteogenesis, and other types of grafts on which implants are then placed but which usually present many serious peri-implantitis issues since the mucosal covering is not adhered gum, are essential for having a set of teeth today. By means of the system object of the present invention, anchorages can be placed on both sides of the defect, and the mandibular arch can be stabilized and restored with the structure or plate, and on it, the set of teeth can be arranged, thereby providing the patient with teeth using an easy technique without risks, and avoiding the complex bone reconstruction techniques discussed above and peri-implantitis, since the connection of the plate with the anchorage is completely covered by the mucosa.

The anchorages of the present system for securing a dental prosthesis are introduced in their entirety in the bone, unlike anchorages of the zygomatic implant having an intermediate portion arranged going through the maxillary sinus without contacting the bone. Therefore, the present system increases the contact surface, thereby increasing osseointegration.

In the present invention, compared to the state of the art of zygomatic implants, the axis for insertion of the anchorage is virtually longitudinal, i.e., from bottom to top, unlike zygomatic implants the axis of introduction of which is angled and on occasion close to horizontality, since they have to be anchored in two different bones, being the jaw bone very atrophic. Therefore, the invention provides better load distribution, eliminating tangential forces, all the forces virtually becoming longitudinal to the major axis of the anchorage, which increases the long-term service life prognosis, reducing the probability of the anchorage breaking. Furthermore, the zygomatic bone is longer along its longitudinal axis than along the oblique or transverse axis, which is where zygomatic implants are currently placed; therefore, longer anchorages can be placed, whereby increasing the osseointegration surface.

In a particular manner, the anchorages can be secured to the maxillary in the horizontal position, going through the bone from wall to wall from its outer face to its inner face. This novel embodiment will be carried out in cases of fine bone ridges to solve the drawbacks of the state of the art, which presents only bone grafts or corticotomies and greenstick fractures as alternatives. In this case, in comparison with zygomatic implants, and even though such implants cannot be applied to solve these cases of fine bone ridge, tangential forces will not occur since, while the primary anchorage area in zygomatic implants is at the distal end thereof, in the portion that is anchored in the zygomatic bone, in the horizontal anchorages of the present invention, the mid two-thirds of the anchorage are in contact with the bone, and the loads are generated on both sides of the bone ridge, and therefore at both ends of the anchorage. The loads are therefore homogenously distributed.

In a particular manner, the anchorages can have in at least one of their ends stabilizing elements, such as nuts and washers, which allows for new uses of the anchorage in cases of thin ridges or insufficient bone height. In the case of fine bone, conventional implants cannot be placed if it is not with bone grafts, or with corticotomy and bone expander techniques causing greenstick fracture of the alveolar bone. In the case of bone height deficiency (<5 mm), as occurs in the subnasal region when there is serious atrophy, or in molar areas due to pneumatization of the maxillary sinus, conventional implants cannot be placed because they do not have primary stability since they are very short implants. In these cases, are essential techniques of maxillary sinus lift and filling or bone grafts to augment bone height. The anchorages of the present invention, with added stabilizing elements, such as a nut and washer, allow from the start enormous primary stability, and therefore the possibility of placing not only the anchorages without grafts or other techniques, but even of placing the teeth immediately.

Preferably, the anchorages of the system for securing a dental prosthesis are formed by a first threaded portion introduced in the bone to which they are going to be secured, and by a second portion for the connection to the plate, said connection being completely covered by the mucosa and a distance from the maxillary alveolar ridge.

The plate, or structure, or mesh, transmits loads from the prosthesis, which is supported on posts, to the anchorages and only to the anchorages, and not to the surface of the bone.

In a particular manner, the plate is made using a biocompatible material, such as titanium, for example, and custom-made by means of CAD/CAM technology, to adapt to the shape of the jaw bone of the patient, and it must be rigid enough so as to not deform and so as to effectively transmit loads of the prosthesis to the anchorages. Unlike former juxta-bone implants, loads are not transmitted to the surface of the bone, causing resorption thereof and failure of the implant, but rather to the anchorage.

In conventional implantology and in the state of the art of zygomatic implants, the posts where the teeth are arranged are directly connected to the anchorages, so there is no possibility of changing the position of said posts. In contrast, in the present invention, the plate is an intermediate element between the posts, where the teeth are arranged, and the anchorages, which allows positioning said posts in the optimal site where must the teeth of the prosthesis must be arranged, thereby achieving better aesthetics and functionality.

In cases of segmental bone defects in the upper or lower jaw, in which a complete bone fragment is missing, the plate runs through the oral cavity, which enables securing the teeth, and furthermore stabilizing the bone ends on both sides of the defect. This is not possible with current means and resources and can only be solved with complex bone reconstruction.

Furthermore, since the plate or structure is custom-made for the patient beforehand by means of CAD/CAM technology or another technology allowing it, it can be available at the time of placing the anchorages, and therefore they can be placed immediately and the prosthesis therefore secured thereto, which could also be done ahead of time.

The posts go through the mucosa that covers the jaw bone in the ideal position for connecting the teeth or dental prosthesis with the plate, and as indicated above, they are not connected directly to the anchorages, but rather to the plate or structure. The posts can be made either integrally with the plate, or they can be detachable from the plate, the plate having threaded holes in this case for screwing in these posts.

Therefore, by means of the present system techniques for immediate placement of dental prosthesis can be implemented by means of posts integrated in the plate, or in two phases, or by means of detachable posts, allowing the anchorages to become osseointegrated and placing the prosthesis after several months.

### Brief Description of the Drawings

An embodiment of the invention making reference to a series of drawings will be described below in an illustrative and non-limiting manner in order to help understand the invention.
Figure 1 is an anterior frontal view of an upper jaw showing the relation of the anchorages of the system object of the present invention with the maxillary sinus, being housed outside of same in the zygomatic bone and longitudinally oriented with the connection in the lower edge of the bone. Figure 1 also shows a horizontal anchorage in the jaw bone for reinforcing the system. The plate adapts to the curvature of the bone but the loads are transmitted to the anchorages, not to the surface of the bone.
Figure 2 is a bottom plan view of the upper jaw of Figure 1, in which the point of insertion and connection of the anchorages to the plate in the zygomatic bone can be seen. Figure 2 also shows the horseshoe shape of the plate and the arrangement of the holes for screwing the posts into the ideal position where the teeth must be arranged.
Figure 3 is a side view of the upper jaw from the preceding figures, where the anchorages in both zygomatic bone and in the upper jaw, and the triangular shape of the plate can be seen. The anchorages are fully contained in a single bone.
Figure 4 is a perspective view of an atrophic lower jaw or mandible. The anchorages are arranged where there is enough bone, and the plate is connected to them, adapted to the shape of the bone and below the mucosa that covers the jaw bone.
Figure 5 is a perspective view of a lower jaw or mandible with a segmental defect. The anchorages are arranged on both sides of the defect and support the plate on which the teeth are secured.
Figure 6 shows the securing of a particular embodiment of anchorages in a jaw bone with thin ridge. The anchorages are placed in the horizontal position completely included in bone, support a saddle plate on which the post is housed for connecting to the tooth. The plate is strongly stabilized by means of a accessory or nut which is screwed onto its distal end.
Figure 7 shows another particular embodiment of anchorages for the securing thereof in insufficient vertical bone. Subnasal localization is presented. The anchorages have a washer and nut, completely stabilizing them even with insufficient bone height.
Figure 8 shows a particular embodiment of a plate with a shape adapted to the upper jaw. Although the plate is adjusted to the surface of the bone, the loads are not transmitted directly to said bone, but rather to the anchorages.
Figure 9 shows an embodiment of a dental prosthesis plate connection post. The dental prosthesis is connected by screwing to the posts.
Figure 10 shows a dental prosthesis plate connection post in the form of a stump, which can be arranged as a separate element screwed to the plate or integral therewith.
Figure 11 shows different examples of an embodiment of a semispherical anchorage. The plate is also semispherical for suitable connection to the anchorages. This semispherical shape allows correcting minor deviations along the axis of insertion of the anchorage.
Figure 12 shows a particular embodiment of an anchorage in which the anchorage is polyhedral. It has a threading worked in its corners for screwing on the accessory which strongly secures the structure to the platform of the anchorage.
Figure 13 depicts an embodiment of a cylindrical anchorage, in which the neck of the platform is polyhedral to allow the insertion of said anchorage into the bone.
Figure 14 shows an embodiment of a mixed cylindrical-polyhedral anchorage. In this case, the cylindrical portion of the anchorage rests on the platform, whereas the polyhedral portion allows insertion of the anchorage into the bone.
Figure 15 shows another embodiment of a mixed polyhedral-cylindrical anchorage. In this case, the polyhedral portion rests on the platform.
Figure 16 shows a securing of the plate to the platform of the anchorages and different combinations thereof by means of different embodiments.
Figure 17 shows an embodiment of an independent part when the plate does not have an outer threading for securing it and it is secured by means of the connection through a threaded rod which is housed in a hole with threading in the upper portion of the anchorage.
Figure 18 shows an example of a flat part in the form of a washer which can be frustoconical or inclined to compensate for minor maladjustments between the anchorage, the plate and the independent part.
Figure 19 shows an example of an anchorage with an internal connection, an example of an independent part for this anchorage and lock screw.

These drawings refer to a set of elements, which are:
1. anchorages
2. oral cavity
3. plate
4. jaw bones
5. extension of the plate for connection to the anchorages
6. posts
7. zygomatic bone
8. first portion of the anchorage to be introduced in the bone
9. second portion of the anchorage for connection to the plate
10. first area of the second portion of the anchorage in the external connection
11. second area of the second portion of the anchorage in the external connection
12. first part of the anchorages of the second group
13. second part of the anchorages of the second group
14. third part of the anchorages of the second group
15. fourth part of the anchorages of the second group
16. first sector of the anchorages of the third group
17. second sector of the anchorages of the third group
18. third sector of the anchorages of the third group
19. nuts
20. washers
21. cover for securing of the plate to the anchorage

### Detailed Description of the Invention

The object of the present invention is a system for securing a dental prosthesis indicated in cases of upper and lower jaw bone loss, insufficient jaw bone, or serious bone atrophy.

As can be seen in the drawings, the system for securing has at least one anchorage 1 in the form of a screw configured for being anchored in a single bone and a distance from the maxillary alveolar ridge, being fully introduced in said bone, and a plate 3 configured for being arranged in the oral cavity 2, under the mucosa that covers the jaw bone 4, in contact with said maxillary or mandibular bone 4, and following the shape thereof and of the mucosa covering it. The plate 3 has an extension 5 whereby it is connected to the anchorages 1, said connection being completely covered by the mucosa and a distance from the maxillary alveolar ridge.

Additionally, the system for securing a dental prosthesis has a plurality of posts 6 arranged in the plate 3 and configured for going through the mucosa that covers the jaw bone 4 in the most optimal position so as to secure the dental prosthesis.

By means of this system, the loads resulting from use of the dental prosthesis are transmitted from the posts to the anchorages 1 through the plate 3, instead of being transmitted to the bone, thereby preventing bone resorption and implant deterioration.

As regards the anchorages 1, these are elements preferably made in the form of a screw which are secured in the bone 4, 7 where they will become osseointegrated in a rigid manner and will serve as support for securing the rest of the required elements, plate 3 and posts 6, in order to secure a dental prosthesis in cases of insufficient or scarcity of bone.

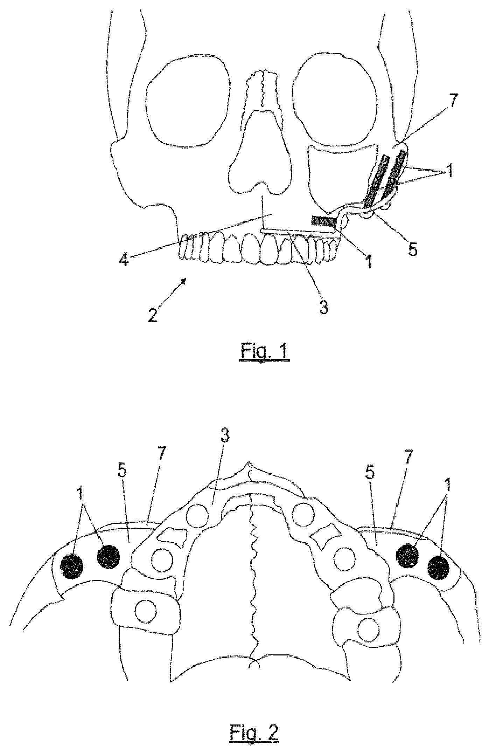

The place where these anchorages 1 emerge, where they are secured to the plate 3 or structure, unlike systems existing in the state of the art, is covered by tissues or mucosa and a distance from the maxillary alveolar ridge, and therefore they do not project directly into the mouth, as can be seen in Figures 1 to 3.

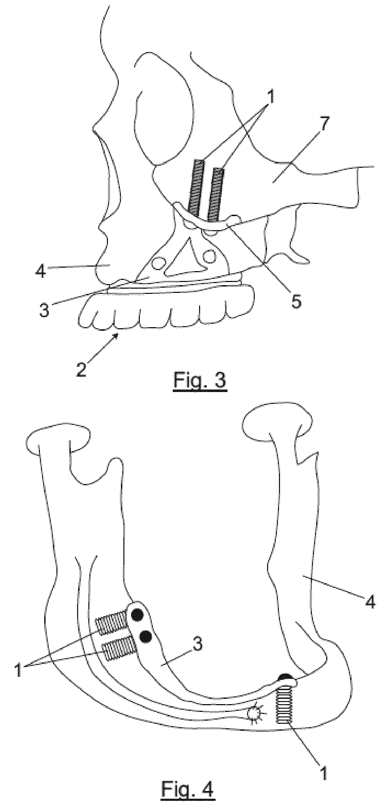

According to different embodiments of the invention, the anchorages 1 are configured for being anchored to the zygomatic bone 7, as can be seen in Figures 1 to 2, from the lower edge thereof and sagittally through same, i.e., from bottom to top, or for being anchored to the upper jaw bone 4, axially through same, i.e., from the outer face to the inner face, also shown in Figure 1, and to the lower jaw bone 4 or mandible, axially, i.e., from the outer face to the inner face through same and sagittally through same from its top edge from top to bottom, as seen in Figure 4.

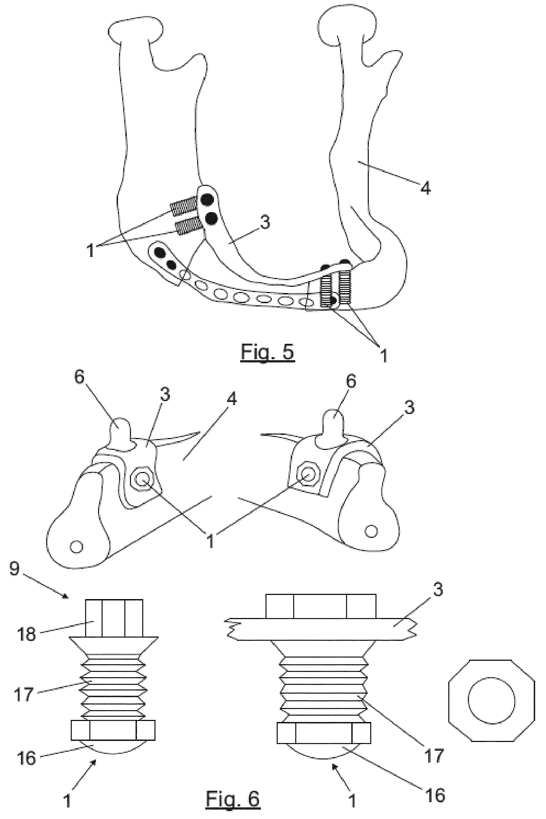

Additionally, in cases of complete loss of a fragment of the lower jaw bone 4 or mandible due to tumor resection or due to trauma, continuity of the mandibular arch is lost and the segments or stumps are spaced from one another on both sides of the defect caused. By means of the system object of the present invention anchorages 1 can be placed on both sides of the defect, and the mandibular arch can be stabilized and restored with the structure or plate 3, and on it, the set of teeth can be arranged, thereby providing the patient with teeth using an easy technique without risks, and avoiding the complex bone reconstruction techniques discussed above. This restoration of the mandibular arch can be seen in Figure 5.

Preferably, the anchorages 1 have at least a first threaded portion 8 introduced in the bone 4, 7 and a second portion 9 for connection to the plate 3.

According to different particular embodiments of the invention, three different groups of anchorages 1 can be distinguished, according to their configuration and use, although they all share the feature that they allow supporting the structure or plate 3 which is what transmits loads to the anchorage 1, and once they are osseointegrated in the bone 4, 7, it is possible and easy to remove the plate 3 if needed due to breakage, deterioration or any other circumstance.

A first group of anchorages 1 is configured for being anchored in the zygomatic bone 7 and in the upper jaw bone 4 or alveolar process, and also in the lower jaw bone 4 or mandible. These anchorages 1 of the first group have two parts or portions:
They have a first portion 8, or body, which is configured to be introduced in the bone 4, 7, and it has a variable shape, generally a frustoconical or cylindrical shape, the distal end thereof being able to end in a blunt or pointed tip. This portion ideally has a threading which allows insertion of the anchorage with rotary motion, and which furthermore stabilizes the anchorage 1 in the bone 4, 7. Furthermore, as an added advantage it can have notches perpendicular to the turns to allow the insertion to be self-tapping, without requiring a threader. This frustoconical shape of the first portion 8 can also not have threads, the distal end ending a flat or semispherical shape for inserting the anchorage 1 by means of impact instead of by means of rotary motion.

A second portion 9 is configured for the connection to the structure or plate 3. According to different particular embodiments, this second portion 9 can have different two ways of being connected, referred to as external connection and internal connection.

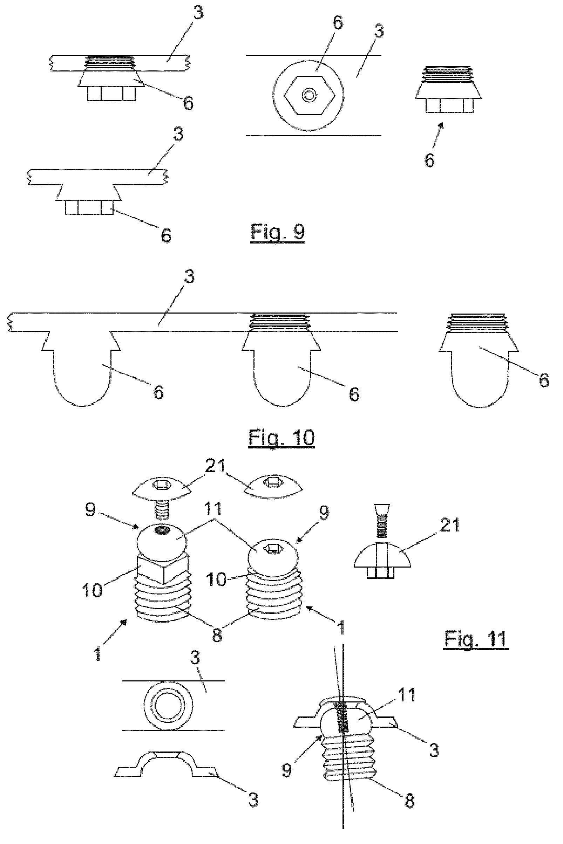

The external connection is depicted in Figures 11 to 15, and the second portion 9 in turn has a first area 10 and a second area 11.

The first area 10 can have a frustoconical shape, can be smooth or with grooves, or a polyhedral shape of a variable height for being able to connect a tool which serves for introducing the anchorage 1 in the bone 4, 7. This first area 10 has a flat platform along the greater diameter of the frustoconical shape.

The second area 11 projects from the platform and consists of a central structure having a variable height and variable spherical, semispherical, polyhedral or cylindrical shape, or having both cylindrical and polyhedral shapes to stabilize the device, or plate 3.

The spherical or semispherical central structure shown in Figure 11 can start directly from the first portion 8 or body of the anchorage 1, or on the first area 10 of the second portion 9. It has a hole in its highest part having inner threading for screwing onto a securing cover 21 securing the plate 3 to the anchorage, and this hole can have a first polyhedral part to make the insertion of the anchorage 1 easier or to improve the connection of the screw. The plate 3 that is secured in this case also has a semispherical shape so that it adapts perfectly to the surface of the anchorage 1 and the advantage of this semispherical shape of both elements is that it allows a few degrees as a margin of error in the placement of the anchorage 1, when it is not placed exactly on the perpendicular to the plate 3 or structure. The screw in this case also has an umbrella shape at its head which adapts to the plate 3, or it can be made up of two or more elements in a variable manner.

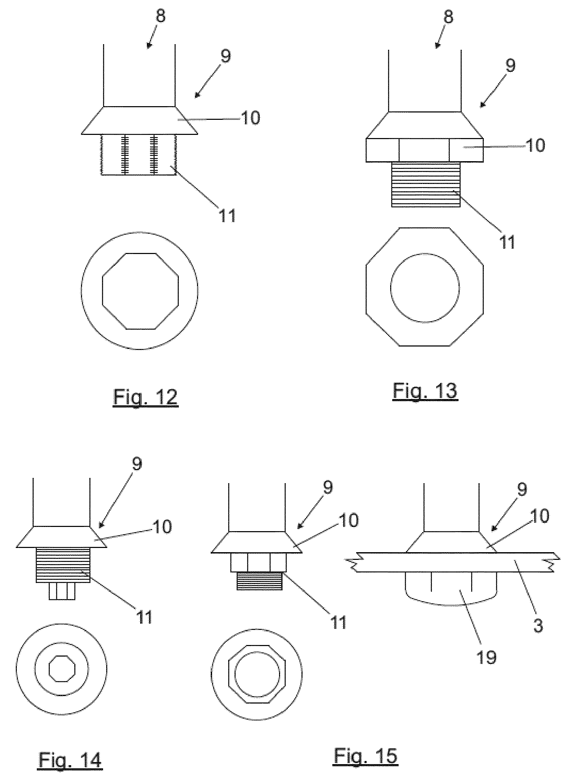

The polyhedral-shaped central structure is depicted in Figure 12, and it is used to allow the rotary motion required for placing the anchorage 1 in the bone 4, 7. This structure has threads or screw threading on the side surface thereof to enable screwing onto an independent part.

The cylindrical-shaped central structure is depicted in Figure 13, and in this case it rests on a first polyhedral area 10 to allow the insertion of the anchorage 1. The cylindrical part may not or may have threading on its entire outer surface to enable screwing onto an independent part.

As regards the combined cylindrical- and polyhedral-shaped central structure, the part cylindrical is arranged on the platform of the first area 10 and a structure polyhedral of variable height is added to said first area 10 to make insertion of the anchorage 1 easier. In this case, the first area 10 has frustoconical instead of polyhedral shape, as seen in Figure 14. Another possibility is the opposite, i.e., the portion resting on the platform is polyhedral, and the cylindrical portion is placed on the portion resting on the platform, as shown in Figure 15. In both cases, the cylindrical part can have a threading on its entire surface to enable screwing on an independent part.

The cylindrical-shaped central structure, polyhedral-shaped central structure or both combined can also be smooth, without threads, and in this case have a hole in the center thereof and in a variable manner have inner threading for the connection of a screw of variable size and shape securing the plate 3 to anchorage 1.

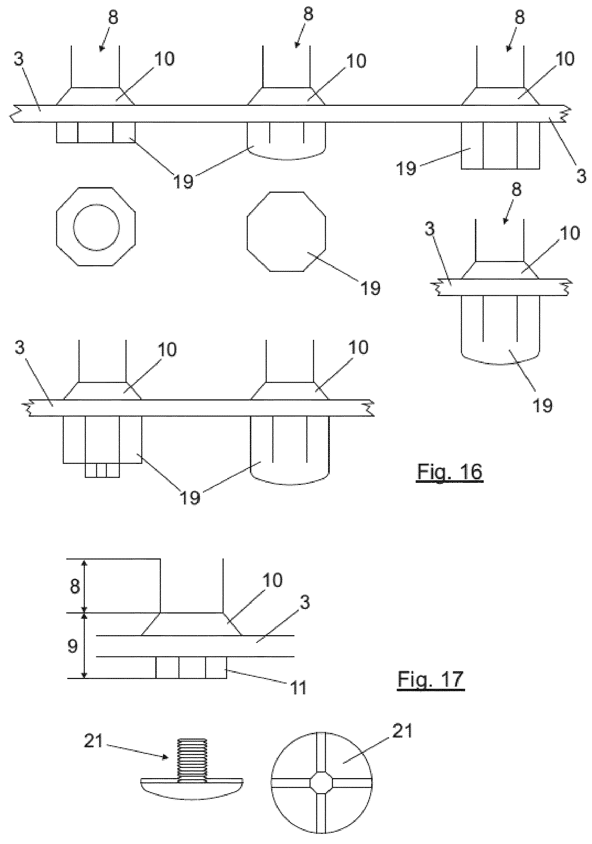

With respect to the independent part that is screwed onto the central structure, as can be seen in Figure 16, it is a polyhedron on its outer face and has threads on its inner face having the same pitch as the central structure onto which it is screwed. The inner diameter of the independent part is equal to the diameter having the threads or threading of the central structure of the second portion 9 of the anchorage.

This independent part can be finished at its end spaced from the platform of the anchorage 1 with an open and flat shape, or with a semispherical shape, being concealed below the central structure.

Figure 17 shows the case of a smooth polyhedral structure without threads, in which the independent part is in the form of a cover with a central threaded rod that is screwed into the central hole of the central structure.

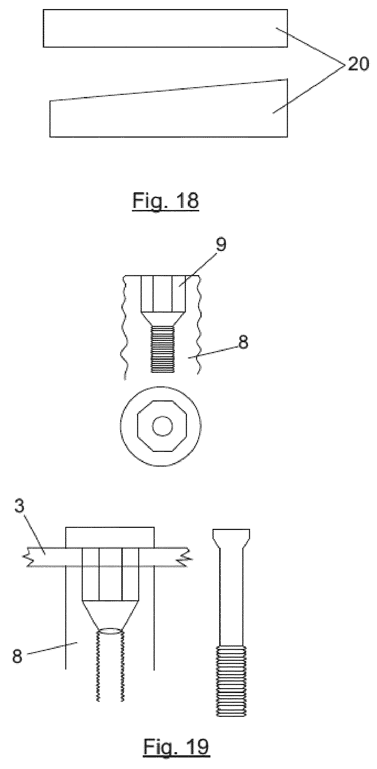

Figure 18 shows an embodiment in which in order for there to be a proper adaptation to the surface of the bone 4, 7, a washer 20 with an asymmetrical shape, with a frustoconical shape, with an oblique section on the face thereof spaced from the base is used. The inclination of the inclined part can be of different degrees to allow better adjustment according to the situation; therefore, small deviations of a few degrees in the longitudinal axis of insertion of the anchorage can be corrected.

Therefore, the assembly formed by the flat platform, the central structure and the independent part, allows the plate 3 supported on the flat platform to be rigidly secured to the anchorage 1, the forces and pressures therefore being transmitted directly to the anchorage and not the bone, the principles of modern of implantology thus being applied.

The internal connection is depicted in Figure 19, and on its surface it has a platform of variable diameter on which the structure or plate rests. On it there is a central hole with a variable frustoconical shape, and in its deepest portion a second hole with inner threading. The frustoconical portion can have flat surfaces or polyhedral notches or not, according to different alternatives. An independent part is secured in said hole.

For the internal connection, the independent part has a cylindrical base having a diameter greater than or equal to the diameter of the anchorage. On this base there is a smooth frustoconical or cylindrical structure and at its end farthest from the base it can have a polyhedral shape with flat faces. This independent part has a hole in the center that goes through said independent part along its entire length and said hole has a diameter similar to that of the hole that the anchorage has in its central part in the case of internal connection. The independent part fits in the hole of the anchorage 1 in the case of an internal connection, although it has a length that is longer than the length of the frustoconical hole of the anchorage 1 and equal to the sum of the length of the frustoconical hole of the anchorage 1 plus the height of the base of the structure or plate for it to be placed between the anchorage 1 and the independent part. This independent part is secured to the anchorage 1 by means of a screw going through it, and it has a length sufficient for being screwed into the hole with inner threading that the anchorage 1 has in its center, the plate 3 thereby being firmly secured.

A second group of anchorages 1 is used to address situations in which there is significant vertical bone loss in the area of the incisors or in the molar areas due to the large maxillary sinus, but in which there is a minimum height of 3 or 4 millimeters. Current techniques for these cases are bone grafts, maxillary sinus lift on two separate occasions (first lift and bone filling and then, 6 months later, implant placement), and alveolar distraction osteogenesis in cases in which there is a minimum height of 10 millimeters but not less. All these techniques used today do not allow the immediate loading of the dental prosthesis; they present morbidity derived from the graft donor area, require a long time for bone healing, and in the case of grafts, a relatively low percentage of complications, such as dehiscences, infection and failure; and in the case of sinus lift, the risk of sinusitis.

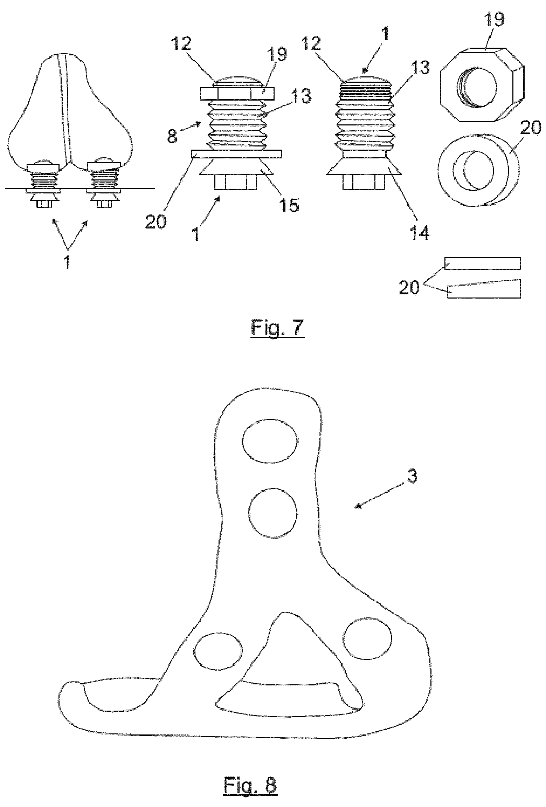

The second group of anchorages 1 of the present invention, shown in Figure 7, solves the complications of the current state of the art, achieving complete primary stability of the anchorages 1, which allows the possibility of immediate loading. If longer anchorages 1 are to be placed for maxillary sinus lift or subnasal mucosa lift, the anchorages 1 of the second group allow the procedures to be performed on a single occasion, with the subsequent savings in time and discomfort, avoiding a second surgery. Furthermore, they can serve to complement the anchorages 1 of the first group for cases of completely edentulous patients requiring a complete upper prosthetic rehabilitation which is secured by screwing.

According to a particular embodiment, these anchorages of the second group usually have four different parts:
The first part 12 or distal part has a cylindrical shape with threads and its distal end is finished with a smooth regular shape so as to not affect the soft tissues. The length can be variable, reduced on one hand to the smallest possible length that allows screwing on an independent part, such as a nut 19, and does not bulge much into the soft tissues, or the desired length can be greater for those cases in which the surgery is to be complemented by sinus and subnasal mucosa lift and bone filling. The independent part used has the same features as the independent part used with the anchorages of the first group, with the distal end rounded and this first part 12 of the anchorage 1 being covered or having an open and flat shape.

The second part 13 or bone part has a frustoconical shape and variable length, and it has threads which can be of variable length, being anchored and fully introduced in the bone. The transition of this second part 13 to the first part 12 can be direct or progressive.

The third part 14, or neck, can be present or not, and it has a smooth cylindrical shape and length reduced to the smallest length possible which allows housing an independent part in the form of a flattened cylinder with a diameter larger than the anchorage 1, such as a washer 20, with a central also cylindrical hole and height equal to the of the neck of the anchorage. Another possibility for there to be proper adaptation to the surface of the bone is for the independent part used to have an asymmetrical shape, such as frustoconical, with an oblique section on its face spaced from the base. The inclination of the inclined part can be of different degrees to allow better adjustment according to the situation.

The fourth part 15, or connection, can be an external or internal connection. In the case of an external connection, it is a frustoconical element which, on its face spaced from the anchorage, is finished with a flat platform on which a polyhedral structure with a central hole with inner threading projects for securing the structure, plate or dental prosthesis. In the case of an internal connection, the face spaced from the anchorage 1 is finished with a central hole having variable frustoconical shape and a second hole with inner threading in its deepest portion, being able to have flat surfaces or polyhedral notches or not in the frustoconical portion to prevent rotation of the dental prosthesis.

In the event that a plate 3 that is attached to other anchorages 1 such as one from the first group is to be secured, the fourth part 15 or connection must be equal to the second portion 9 of the anchorages 1 of the first group, and therefore it must also contain the elements described above, as an independent part, adapting said part according to whether the connections are the so-called internal connection and external connection.

According to an alternative embodiment of these anchorages of the second group, said anchorages only have three parts, lacking the third part 14 described above.

The third group of anchorages 1 is applied primarily to the upper jaw bone 4 and to the lower jaw bone 4, or mandible. The fundamental use of these anchorages 1 of the third group is in cases in which there is horizontal bone deficiency or thickness deficiency, but not vertical bone deficiency. In these cases, the alveolar process or ridge are thin, with a triangular shape when viewed in cross-section. These anchorages 1 of the third group are placed in a horizontal plane, fully traversing the thickness of the bone from its outer face to its inner face 4 and allow supporting a "saddle" plate 3 which is adapted to the shape of the bone 4 and on which the dental prosthesis rests. Current techniques for solving this issue of horizontal bone deficiency are bone grafts, and as discussed above, these have a relatively high percentage of complications, such as dehiscences, infection and failure, especially in the lower jaw bone, or mandible. These anchorages 1 of the third group are depicted in Figure 6.

The anchorages 1 of the third group have three different sectors:
The first sector 16, or apical sector, is equal to the first part 12 of the anchorages of the second group.
The second sector 17, or sector bone, is equal to the second part 13 of the anchorages of the second group.
The third sector 18, or connection, is equal to the second portion 9 of the anchorages of the first group.

The plate 3 is preferably made using a biocompatible material and custom-made for the patient by means of CAD/CAM technology, so as to be able to adapt to the shape of the jaw bone 4. The material can be, for example, titanium, without prejudice to other biomaterials with sufficient rigidity and hardness to support a dental prosthesis and the loads and forces it must absorb in the chewing function. The plate 3 perfectly adapts its shape to the bone surface on which it rests, but is not supported, given that the plate 3 is supported on the anchorages 1, not on the bone 4, 7. The plate is positioned such that it is covered or below the oral mucosa that covers the jaw bone 4.

The design of the plate 3 can vary in shape and in thickness according to load requirements that must be withstood.

The shape and profile of the plate 3 is customized for each case and range from a linear shape for situations of having to replace one or two teeth, a triangular shape for more parts, or half a jaw bone, a horseshoe shape for complete upper jaw bone prostheses, or other shapes as needed. Different embodiments of shapes of plates 3 can be seen in Figures 1, 2, 3, 8 and 6.

The optimal number of anchorages 1 for suitable securing of the plate 3 obviously varies according to each particular clinical situation. In general, one or two anchorages 1 should be placed in the zygomatic bone 7 and one or two anchorages 1 going through the jaw bone 4 from the outside in, i.e., from the outer face to the inner face of the alveolar portion of the bone to provide greater stability.

In the case of the lower jaw bone 4, or mandible, when there is a complete absence or serious atrophy of bone in the mandibular body (pre-molar and molar areas), the plate 3 would have a fundamentally rigid shape, i.e., it would follow the profile of the bony ridge of the gum. In these cases, anchorages 1 can be placed distally in the area of the retromolar trigone or even in the ascending branch, and proximally where there is enough bone in front of the exit of the dental nerve in the mental foramen. The number of anchorages 1 required for these mandible cases can vary for each clinical situation, but in general two anchorages should be placed in the distal area and one or two in the proximal area, as can be seen in Figure 4.

In cases of segmental mandible defects (complete loss of a segment) that has not been reconstructed with free or microvascularized bone grafts, as can be seen in Figure 5, the structure or plate 3 must traverse the oral cavity like a bridge between both segments. In these cases, one reconstruction plate 3 of the many existing in the basal area in both mandible stumps should be used to reconstruct the mandibular arch and stabilize both fragments.

In the use of the system for securing in cases in which there is horizontal bone deficiency or thickness deficiency, but not vertical bone deficiency, in which the plate 3 is "saddle"-shaped, as can be seen in Figure 6, said plate 3 is adapted to the outer or vestibular face and to the inner face called the lingual or palatine face. The stump or post 6 on which the tooth is secured, which is the part that goes through the mucosa to emerge into the mouth is incorporated on the plate in the highest ridge or higher portion thereof. In case of several teeth, the plate 3 extends as far as required, one or more anchorages 1 that go through the bone 4 from the outer part to the inner part being able to be placed for any case and as needed. The plate 3 would have polyhedralshaped holes to prevent rotations of the anchorage 1 and they are secured to the anchorage 1 by means of the independent parts described above, thereby achieving the stability required even for use in immediate loading.

In relation to the posts 6 of the system for securing, and as can be seen in Figures 9 and 10, the dental prosthesis rests on these posts and these posts can be made integrally with the plate 3, for the immediate placement of the dental prosthesis, or they can be detachable from the plate 3, the plate 3 in this case having threaded holes for screwing onto said posts 6, for the placement of the dental prosthesis at a subsequent time after osseointegration of the anchorages 1.

The posts 6 are the elements of the system that go through the mucosa in order to connect the teeth or prosthesis to the plate 3, and since they are not connected directly to the anchorages 1, they allow positioning them in the optimal site to achieve better functionality and aesthetics. They can have different shapes according to different embodiments:
Threaded universal platform: this is a post 6 that reproduces the shoulder and platform of conventional implants on the market and is screwed to the plate 3 by means of a rod threaded in its lower part, as shown in Figure 9.

Universal platform integrated in the structure: this is a post 6 similar to the one described in the preceding case, but it is not an isolated post 6, rather it is fused or welded to the plate 3, as can also be seen in Figure 9.

Screwed post for cemented prosthesis: this is a post 6 for the direct cementing of teeth and it is screwed to the plate 3 by means of a rod threaded in its lower part, as depicted in Figure 10.

Post integrated in the structure: similar to the foregoing, but it is not an isolated post 6, but rather it is integrally fused or welded to the plate 3, as can also be seen in Figure 10.

Having clearly described the invention, it is hereby stated that the particular embodiments described above are not limiting and are susceptible to modifications in detail provided that they do not alter the fundamental principle and essence of the invention.

## Claims

1. A system for securing a dental prosthesis, **characterized in that** it comprises
- at least one anchorage (1) in the form of a screw configured for being anchored in a single bone a distance from the maxillary alveolar ridge, being fully introduced in said bone,
- a plate (3) configured for being arranged in the oral cavity (2), under the mucosa that covers the jaw bone (4), in contact with said jaw bone (4), and following the shape thereof and of the mucosa covering it, comprising an extension (5) whereby said plate (3) is connected to the anchorages (1), said connection being fully covered by the mucosa and a distance from the maxillary alveolar ridge,
- and a plurality of posts (6) arranged in the plate (3) and configured for going through the mucosa that covers the alveolar ridge of the jaw bone (4) so as to secure the dental prosthesis,
the loads resulting from the use of the dental prosthesis being transmitted to the anchorages (1) through the plate and the posts, where the dental prosthesis (3) rests.

2. The system for securing a dental prosthesis according to claim 1, **characterized in that** the anchorages (1) comprise in at least one of their ends stabilizing elements selected from nuts (19) and washers (20).

3. The system for securing a dental prosthesis according to any of the preceding claims, **characterized in that** the anchorages (1) are configured for being anchored to the zygomatic bone (7), from its lower edge on the outer face and in a sagittal axis from bottom to top through same.

4. The system for securing a dental prosthesis according to any of claims 1 to 2, **characterized in that** the anchorages (1) are configured for being anchored to the upper jaw bone (4) and to the lower jaw bone (4), in both cases a distance from the alveolar ridge of said jaw bones (4).

5. The system for securing a dental prosthesis according to any of the preceding claims, **characterized in that** the anchorages (1) comprise
- a first portion (8) selected from a threaded portion and smooth portion introduced in the bone (4, 7), and
- a second portion (9) for connection to the plate (3).

6. The system for securing a dental prosthesis according to any of the preceding claims, **characterized in that** the plate (3) is made using a biocompatible material.

7. The system for securing a dental prosthesis according to any of the preceding claims, **characterized in that** the plate (3) is custom-made by means of CAD/CAM technology for being adapted to the shape of the jaw bone (4).

8. The system for securing a dental prosthesis according to any of the preceding claims, **characterized in that** the posts (6) are integrated in the plate (3).

9. The system for securing a dental prosthesis according to any of claims 1 to 7, **characterized in that** the posts (6) can be removed from the plate (3), and **in that** said plate (3) comprises a plurality of threaded holes for screwing in the posts (6).
